(19) Europäisches Patentamt
European Patent Office
Office européen des brevets

(11) **EP 3 146 452 B1**

(12) **EUROPEAN PATENT SPECIFICATION**

(45) Date of publication and mention
of the grant of the patent:
**01.04.2020 Bulletin 2020/14**

(51) Int Cl.:
***G09F 19/20*** *(2006.01)*    ***G16H 50/50*** *(2018.01)*
***G16H 50/20*** *(2018.01)*

(21) Application number: **15731106.9**

(22) Date of filing: **12.05.2015**

(86) International application number:
**PCT/IB2015/053476**

(87) International publication number:
**WO 2015/177680 (26.11.2015 Gazette 2015/47)**

(54) **METHOD AND SYSTEM FOR GUIDING PATIENT SELF-CARE BEHAVIORS**

VERFAHREN UND SYSTEM ZUR ANLEITUNG VON PATIENTENSELBSTPFLEGEVERHALTEN

PROCÉDÉ ET SYSTÈME POUR GUIDER DES COMPORTEMENTS DE SOINS AUTONOMES DE PATIENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.05.2014 US 201462000031 P**

(43) Date of publication of application:
**29.03.2017 Bulletin 2017/13**

(73) Proprietor: **Koninklijke Philips N.V.**
**5656 AG Eindhoven (NL)**

(72) Inventors:
• **NIKOLOVA-SIMONS, Mariana**
**NL-5656 AE Eindhoven (NL)**
• **LEE, Matthew Len**
**NL-5656 AE Eindhoven (NL)**

(74) Representative: **Drapeau-Paquin, François**
**Philips International B.V.**
**Intellectual Property & Standards**
**High Tech Campus 5**
**5656 AE Eindhoven (NL)**

(56) References cited:
**WO-A2-01/09759**        **WO-A2-2013/098719**
**US-A1- 2011 153 350**    **US-A1- 2012 030 156**

**Description**

**Background**

Patients suffering from chronic medical conditions may be required to perform self-care behaviors in order to manage their conditions and improve their clinical outcomes. However, individual patients may have varying determinants that influence their ability to comply with self-care behaviors, and it may be desirable to address and improve these determinants as part of a care plan in a patient-specific manner. Further, patients may not always understand the specific manner in which self-care behaviors affect their clinical outcomes, which may cause them not to comply with their self-care behaviors. One example of prior art patent document dealing with this problems is WO 2013/098719 A2.

**Brief Description Of The Drawings**

Figure 1 schematically illustrates a system for generating a patient-specific ordered list of determinants according to an exemplary embodiment.

Figure 2 illustrates an exemplary method by which the system illustrated in Figure 1 may operate.

Figure 3 illustrates a table of self-care determinants that may be considered during the performance of the method of Figure 2.

Figure 4A illustrates an exemplary result of the performance of step 210 of the method of Figure 2.

Figure 4B illustrates an exemplary result of the performance of step 220 of the method of Figure 2.

Figure 4C illustrates an exemplary result of the performance of step 230 of the method of Figure 2.

Figure 4D illustrates an exemplary result of the performance of step 240 of the method of Figure 2.

Figure 4E illustrates an exemplary result of the performance of step 250 of the method of Figure 2.

Figure 4F illustrates an exemplary result of the performance of step 260 of the method of Figure 2.

Figure 4G illustrates an exemplary result of the performance of step 270 of the method of Figure 2.

Figure 5 schematically illustrates an exemplary system for informing patients about the effects that performance of self-care behaviors may have on their health.

Figure 6 illustrates an exemplary method by which the system illustrated in Figure 5 may operate.

Figure 7 illustrates an exemplary patient profile that may be generated by the method of Figure 6.

Figure 8 illustrates a portion of an exemplary database that may be used by the system of Figure 5 during the performance of the method of Figure 6.

Figure 9 illustrates a table showing adjustment of the value of patient self-care behaviors according to patient's self-management abilities according to the method of Figure 6.

Figure 10 illustrates a table showing adjustment of the value of patient self-care behaviors according to input parameters according to the method of Figure 6.

Figure 11 illustrates an exemplary user interface display that may be generated by the system of Figure 5.

**Detailed Description**

The exemplary embodiments may be further understood with reference to the following description and the related appended drawings, wherein like elements are provided with the same reference numerals. Specifically, the exemplary embodiments relate to methods and systems for prioritizing patient self-care behaviors and providing patients with support in choosing such behaviors. The invention is defined by the appended claims.

Increasing numbers of patients worldwide suffer from chronic conditions that are associated with poor outcomes such as diminished quality of life, frequent hospital readmissions, and early mortality. Self-care is useful for avoiding these poor outcomes, and, thus, is frequently recommended to patients as part of the treatment of chronic conditions. The effectiveness of such treatment relies on patients' adherence to self-care behaviors such as taking medication, maintaining an appropriate level of physical activity, consuming appropriate types and quantities of foods and beverages, cessation of smoking, managing symptoms, etc. Non-adherence to self-care behaviors is a leading cause of exacerbation of chronic conditions and poor outcomes. Therefore, it is desirable to provide patients with proper interventions to enable effective adherence to self-care behaviors, and to demonstrate to patients the effects of adherence to self-care behaviors.

Each patient will have various determinants that affect the patient's ability to adhere to self-care behaviors. Determinants may include factors such as disease burden, perceived control, self-efficacy, social support, ability to cope with problems, anxiety, depression, willingness to self-manage, comfort in a group, computer skills, etc. The patient's strength or weakness in each of the above determinants may help or hinder the patient's efforts to adhere to self-care behaviors such as those discussed above. Because this is the case, a patient's care plan ("CP") may include efforts to address the patient's determinants and thereby improve the likelihood that the patient will adhere to self-care behaviors. For example, a patient coping with anxiety or depression may be instructed to undergo counseling; a patient with poor computer skills may not be offered an Internet-based behavior change program.

Any given chronic condition may have corresponding weights for various self-care behaviors; for example, for a patient suffering from lung cancer, smoking cessation may be a pivotal factor, whereas the same self-care behavior may be less significant for a patient having a non-cardiopulmonary ailment. Further, any given patient will have some determinants that are areas of strength for the patient, and others that are areas of weakness. Additionally, there may typically be a limited amount of time available in which to address a given patient's determinants. Therefore, it may be desirable to have a patient-specific prioritized list of determinants in order to guide clinicians in designing a CP for the patient. The exemplary embodiments described herein may generate such a patient-specific prioritized list of determinants.

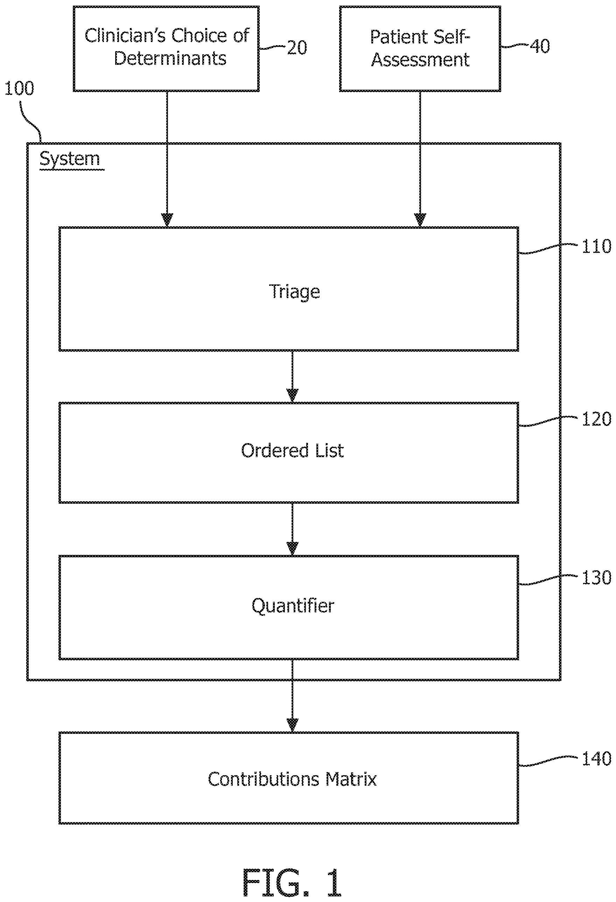

Figure 1 illustrates, schematically, an exemplary system 100 for generating a patient-specific ordered list of determinants. It will be apparent to those of skill in the art that the system 100 includes data processing elements that may be implemented through a combination of hardware (e.g., processor, memory, user interface, etc.) and software in a manner that will be understood by those of skill in the art. The operation of the system 100 will be described herein with reference to the exemplary method 200.

The system 100 may include a Triage element 110. The Triage 110 may perform a first portion of the tasks performed by the system 100. In step 210, the Triage 100 may receive, as input, a clinician's choice of determinants 20 to be used in assessing the patient. The clinician may choose a subset of all possible determinants discussed above (e.g., disease burden, perceived control, self-efficacy, social support, coping with problems, willingness to self-manage) for use in assessing the patient. The choice may be based on clinical guidelines, results of clinical trials, the clinician's experience, etc. For example, a clinician may choose to exclude "computer skills" from consideration because a patient's low level of computer skills may indicate that a telehealth system may not be an efficient use of resources for the patient; those of skill in the art that this reason is only exemplary and that various other reasons for excluding a determinant for consideration may be possible. The subset of determinants retained for subsequent analysis will be referred to hereinafter as $D_{mod}$, meaning modifiable determinants.

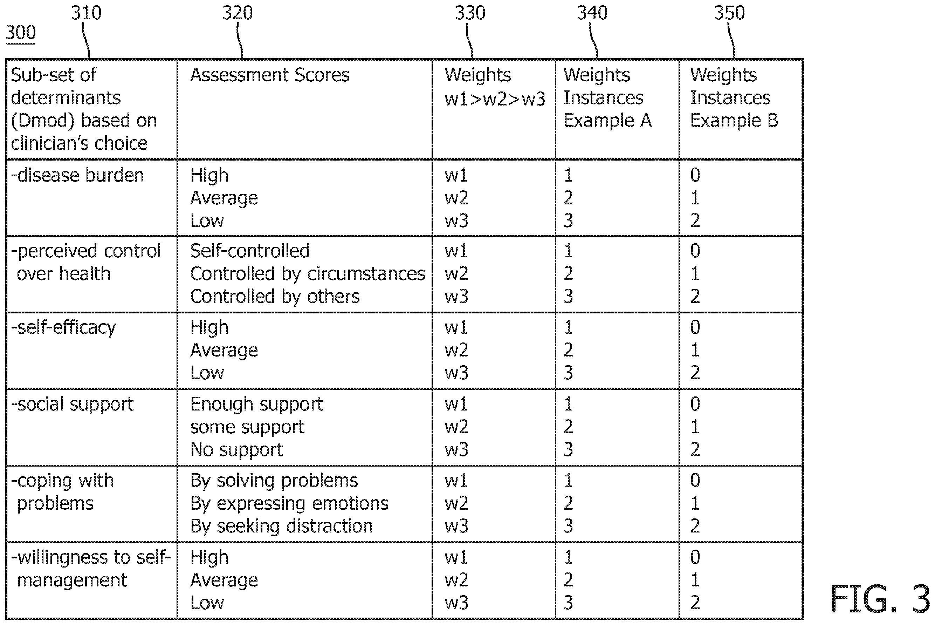

In step 220, the Triage 110 may also receive, as an input, data from a patient self-assessment 40. The self-assessment 40 may be performed using a known assessment tool, such as Self-Management Assessment ("SeMaS") or Patient Activation Measure ("PAM") questionnaires. As a result of this self-assessment 40, each determinant may be assigned a score which classifies a determinant as a facilitator or barrier of self-care behavior. Figure 3 illustrates a table 300 that shows, in a first column 310, the list of determinants selected by the clinician as the applicable subset of determinants $D_{mod}$, and, in a second column 320, possible assessment scores for each selected determinant based on the patient self-assessment 40. Based on these scores, each deteterminant may be classified as either a facilitator or a barrier; for example, high self-efficacy may be classified as a facilitator, whereas low self-efficacy may be classified as a barrier.

Based on the scores for the determinants $D_{mod}$ included in the table 300 of Figure 3, in step 230 the Triage 110 assigns a weight to each determinant in the set $D_{mod}$. Weights may be assigned according to the rule that a determinant with an assessment score indicating that the determinant is a facilitator of self-care behavior is assigned a low weight, while a determinant with an assessment score indicating that the determinant is a barrier to self-care behavior is assigned a high weight. This may indicate that it is more important for a CP to focus on barriers to self-care than on facilitators. The specific quantitative weights used in this step may vary among differing embodiments.

Column 330 of table 300 illustrates generalized versions of weights that may be used herein. Column 340 of table 300 illustrates a first exemplary weighting that may be applied by the Triage 110. In the first exemplary weighting, scores are given weights from 1 to 3, with facilitators scored a 1 and barriers scored a 3. Column 350 of table 300 illustrates a second exemplary weighting that may be applied by the Triage 110. In the second exemplary weighting, facilitators are scored a 0 and barriers are scored a 2. It will be apparent to those of skill in the art that, by weighting facilitators a 0, they may be excluded from further consideration in the modification of self-care behavior.

In step 240, the Triage 110 creates an ordered list 120 of determinants of self-care behaviors based on the weights assigned in step 230. This may involve ranking, by weight, the determinants $D_{mod}$ that remain under consideration after any potential elimination from consideration of some of the determinants due to being assigned zero weight in step 230. This ordered list 120 of self-care determinants may be the output generated by the Triage 110. In step 250, the ordered list 120 is passed, by the Triage, to Quantifier element 130 of the system 100.

In step 260, the Quantifier 130 calculates a weight $W_D$ of each determinant from step 240 across all determinants selected for inclusion in the CP for the patient by virtue of being selected in step 210 and not assigned zero weight in step 230. The weight for $W_D$ each determinant is calculated as:

$$W_D\left(D_{mod}\left(i\right)\right) = \frac{weight\left(D_{mod}\left(i\right)\right)}{\sum weight\left(D_{mod}\left(i\right)\right)}, \quad i=1,\ldots,\#D_{mod}$$

In the above expression, "$\#D_{mod}$" is the quantity of remaining determinants in the set $D_{mod}$. In step 270, the Quantifier 130 calculates the determines a contributions matrix 140, in which the contribution c(i,j) of each determinant i from step 240 to the care plan for a particular self-care behavior j based on the weights $W_D$ calculated in step 260 and known weights $W_B(j)$ for each behavior. The contributions c(i,j) may be calculated as:

$$c\left(i,j\right) = W_D\left(D_{mod}\left(i\right)\right) * W_B\left(j\right), \quad i=1,\ldots,\#D_{mod}; \quad j=1,\ldots,\#Behaviors$$

In the above expression, "#Behaviors" is the number of self-care behaviors under consideration. After step 270, the method 200 is complete. The set of contributions c(i,j) of the contributions matrix 140 output by the Quantifier 130 in step 270 may be the output of method 200 performed by system 100. The matrix 140 of contributions c(i,j) is patient-specific based on the patient's self-assessment 40, and may then be used by a clinician in devising a CP to enable important determinants to be addressed, and, in turn, to enable the patient to adhere to important self-care behaviors. It will be apparent to those of skill in the art that the division of the functions of the Triage 110 and Quantifier 130 described herein may essentially be a logical construct. Thus, they may be integrated into a single element (e.g., a software application, combination of software and hardware, etc.) without departing from the broader scope of the functions described.

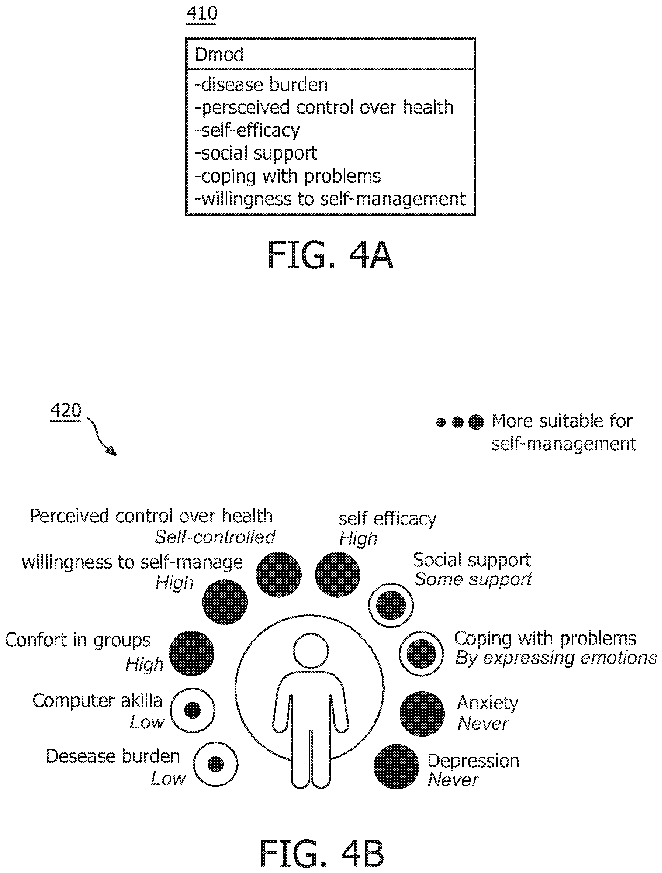

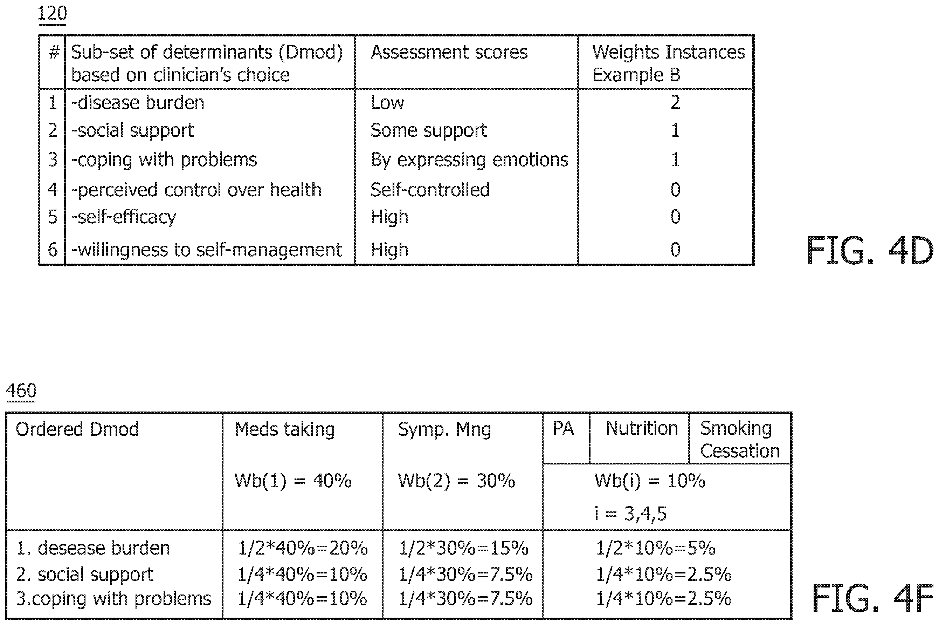

Figures 4A-4G illustrate the results of various phases of the application of method 200 to one exemplary patient. For the condition of the patient who is the subject of these figures, the behavior weight $W_B$ for medication taking is 40%, $W_B$ for symptom management is 30%, $W_B$ for physical activity is 10%, $W_B$ for nutrition is 10%, and $W_B$ for smoking cessation is 10%, as indicated in Figure 4F. Figure 4A illustrates a set of determinants $D_{mod}$ 410 that may be received from a clinician for the patient in step 210. As noted above, this set of determinants may be selected by the clinician based on the patient's condition and various other factors. Figure 4B illustrates the results of the patient's self-care assessment 420. The results shown in Figure 4B are formatted in accordance with the SeMaS assessment tool, and it will be apparent that similar information may be where the patient's self-care assessment has been made using a different technique. In Figure 4B, determinants with large dots are areas of strength for the patient, and will be termed facilitators of self-care, while determinants with small dots are areas of weakness and will be termed barriers.

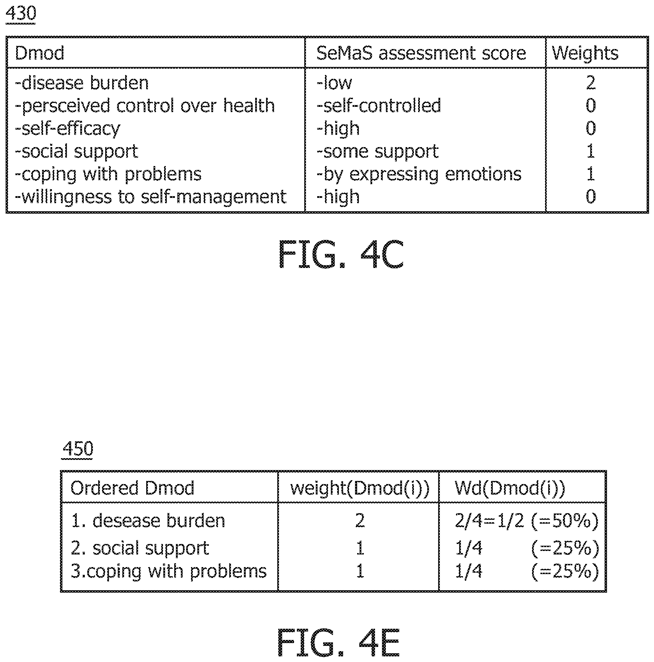

Figure 4C illustrates a weighted set of determinants $D_{mod}$ 430 that may be determined by the Triage 110 in step 230 based on the inputs shown in Figures 4A and 4B. The determinants 430 are weighted as shown in column 350 of Figure 3, described above. Figure 4D illustrates an ordered list of determinants Dmod 120 that may be determined by the Triage 110 in step 240, and may be the output of the Triage 110 to the Quantifier 130.

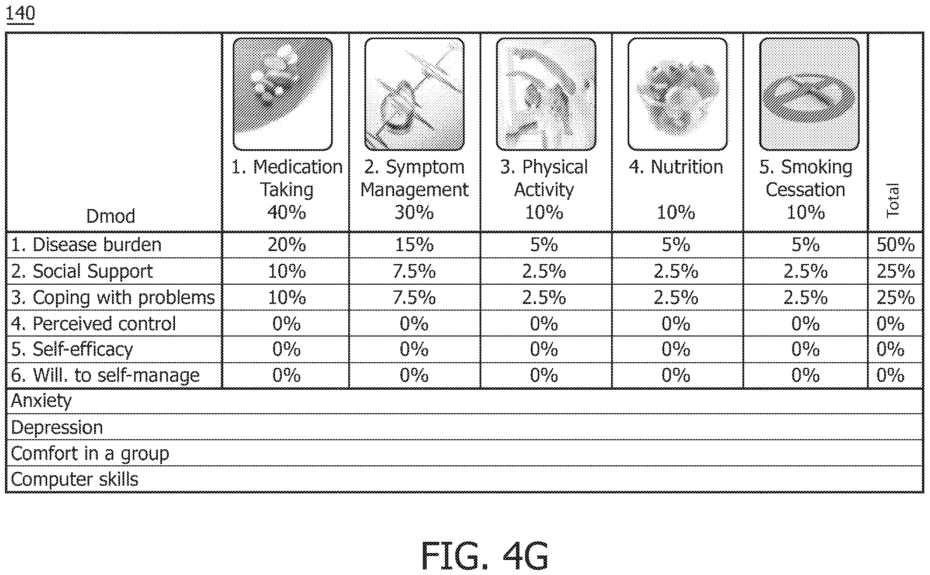

Figure 4E illustrates a set of weights $W_D$ 450 of each determinant from step 240 (e.g., shown in Figure 4D), as divided in step 260 by the Quantifier 130 across all determinants selected for inclusion in the CP for the patient. Figure 4F illustrates a table 460 showing the calculation of the contributions of the patient's determinants to the self-care behaviors appropriate for the patient's condition. Figure 4G illustrates a contributions matrix 140 that may be determined by the Quantifier 130 in step 270 of the method 200, based on the inputs described above with reference to Figures 4A-4F. The contributions matrix 140 may be used by a clinician to determine a CP for the patient having these inputs.

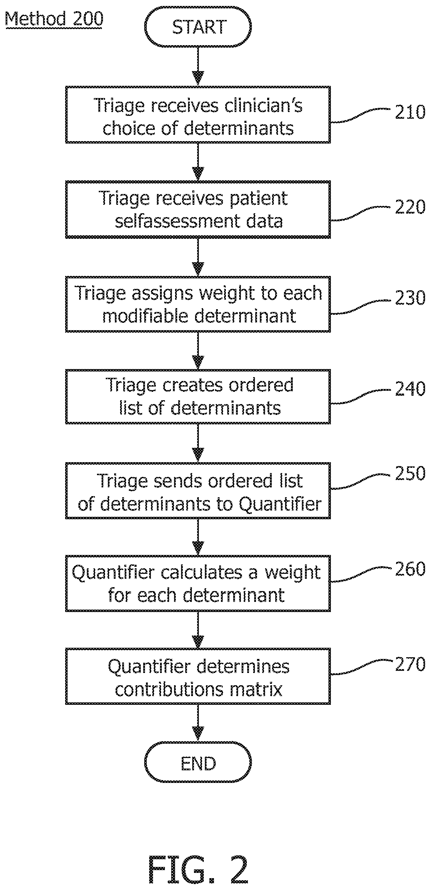

As described above, self-care behaviors are an important factor in controlling the progression of chronic diseases and supporting overall wellness. The exemplary embodiments described above with reference to Figures 1, 2, 3 and 4A-4G present techniques by which clinicians may evaluate a patient's determinants (e.g., factors that may influence the patient's ability to adhere to self-care behaviors), and use the evaluation of determinants to shape a care plan that may enable the patient's determinants to be addressed in a manner that may improve the likelihood that the patient will adhere to self-care behaviors. However, other applications for the patient's ordered list of determinants exist. The exemplary embodiments discussed hereinafter present exemplary embodiments providing another application therefor.

One issue with self-care behaviors is that, although clinicians have a variety of ways of selecting an effective self-care behavior for a given patient, patients themselves may have difficulty integrating self-care behaviors into their daily lives. In part, this may be because it is difficult for patients to understand the level of effort that may be appropriate for a given self-care behavior, and the potential impact that such effort may have on their health. Because of this lack of understanding, patients may be discouraged from adopting new self-care behaviors into their lives. Further, a patient may find it too difficult to adopt a self-care behavior with parameters that are too challenging (e.g., an exercise regimen that is too long or performed too often).

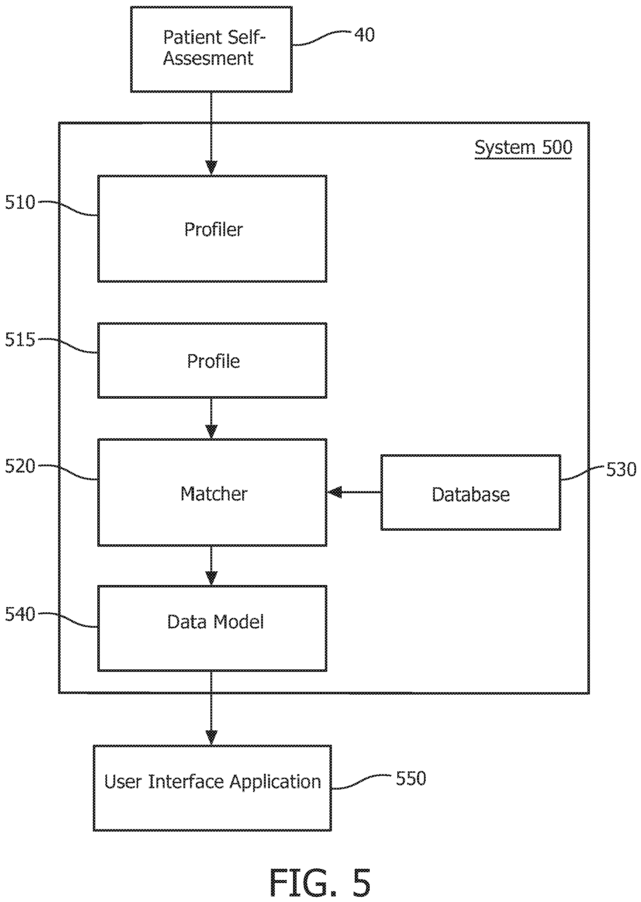

Figure 5 illustrates schematically an exemplary system 500 that may enable patients to more clearly understand the effect that self-care behaviors have on their health. More specifically, the exemplary system 500 may apply patient-specific parameters to general information about a condition to provide a patient with an interactive tool that may enable the patient to understand how the patient's selected manner of performing self-care behaviors affect the patient's health. The system 500 may consist of logical elements performing different tasks, in the same manner as the system 100 described above, but those of skill in the art will understand that the functions of these elements may be grouped together in other embodiments. The operation of the system 500 will be described herein with reference to the exemplary method 600.

The system 500 may include a Profiler element 510. In step 610, the Profiler 510 retrieves available data about a specific patient. The data retrieved by the Profiler 510 may include results of assessment questionnaires about the patient's condition, data about the patient's self-management factors, data about the patient's communication style, data about the patient's previous clinical experiences, and data about the patient's personal preferences. It will be apparent to those of skill in the art that the specific data retrieved by the Profiler may vary among differing embodiments, and that the specific sources consulted may also vary. In one embodiment, a source of the data retrieved in step 610 may be the self-assessment 40 referred to above with reference to Figure 1.

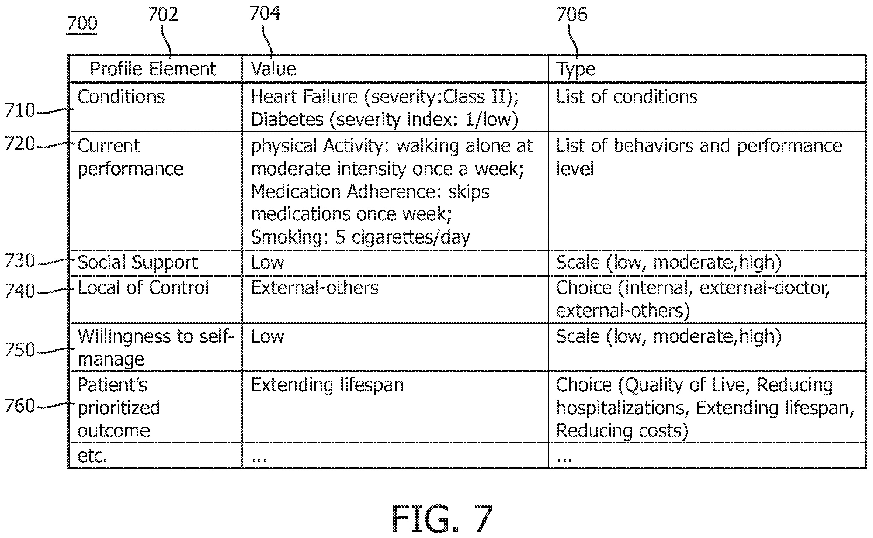

Based on the data received in step 610, in step 615 the Profiler 510 creates a profile 515 for the patient. Figure 7 illustrates an exemplary profile 700 that may be created in step 610. The profile 700 of Figure 7 includes a profile element column 702 that describes the contents of each row, a value column 704 that contains the patient's value for the given row, and a type column 706 that describes the type of information contained in each row. The profile 700 includes conditions 710, describing the conditions being experienced by the patient. The exemplary patient of the profile 700 suffers from heart failure and diabetes. The profile 700 also includes current performance 720, describing current self-care behaviors of the patient. The exemplary patient of the profile 700 exercises once a week, skips medications once a week, and smokes five cigarettes per day. The profile 700 also includes social support 730, describing the level of social support available to the patient.

The profile 700 also includes locus of control 740, describing the extent to which individuals believe they themselves can control events that affect their health (e.g., internal locus of control) or that others have the main control of events that affect the individual (e.g., external locus of control). The exemplary patient of the profile 700 has an external-others locus of control. The profile 700 also includes willingness to self-manage 750, describing the patient's willingness to self-manage his/her condition. The exemplary patient of the profile 700 has low willingness to self-manage. The profile 700 also includes patient's prioritized outcome 760, describing the patient's outcome priority. The prioritized outcome 760 of the exemplary patient of the profile 700 is extending lifespan. It will be apparent to those of skill in the art that the profile 700 is only exemplary. The specific rows shown in the profile 700 may vary among differing embodiments, and the specific values shown in the profile 700 are only one possible patient example. The profile 700 may be stored as a computer file in any format appropriate for subsequent use as will be described hereinafter. The profile 700 may also be exported in a computer-friendly format such as XML, or in a human-readable format for display, printing, etc.

The system 500 also includes a Matcher component 520 in communication with the Profiler 510 and a Database 530. The Database 530 stores self-care behaviors (e.g., physical activity, smoking cessation, etc.) indexed by various factors such as associated conditions, evidence of effectiveness, guidelines and recommended care plans, and population-generalized weights of the individual parameters of a behavior. In particular, the Database 530 may include, for each self-care behavior, a generalized (e.g., not patient-specific) ImpactScore for each medical condition, based only on known scientific evidence and expert opinion.

An ImpactScore quantifies the effect that a self-care behavior has on a given medical condition; the effect that self-care behavior b has on medical condition m will be referred to herein as $ImpactScore_{bm}$. All ImpactScores contained in the Database 530 may be represented on a common scale; in one exemplary embodiment, this scale may be from -100 to 100, but it will be apparent to those of skill in the art that the specific scale used may vary. For example, the physical activity self-care behavior may be associated with multiple medical conditions. For conditions for which scientific evidence and expert opinion have found to be impacted positively by physical activity, a high weight (e.g., an ImpactScore of 85 on a scale from -100 to 100) may be associated with them; for those with less evidence of positive impact, a lower weight (e.g., an ImpactScore of 30 on the same scale); for those conditions where a negative impact is found, a negative weight may be designed (e.g., an ImpactScore of -20 on the same scale).

The Database 530 may also include a set of parameters describing how each self-care behavior is performed. For example, the self-care behavior or physical activity may have a parameter called "type" that may take on semantic values such as walking on a treadmill at home, attending a fitness class, walking at the mall, or following a fitness DVD at home. Another parameter may be "intensity," which may take on semantic values such as low, moderate, or high. Another parameter may be "frequency," which may take on values such as once per week, twice per month, three times a day, etc. It will be apparent to those of skill in the art that the specific parameters may vary for each self-care behavior contained in the Database 530, depending on the nature of the self-care behavior. The Database 530 may also include a weight for each parameter (e.g., ranging from 0 to 1.0) describing the parameter's relative contribution to the effectiveness of the behavior, based on scientific evidence or expert opinion. The Database 530 may also include a recommended configuration of the parameters for each behavior and medical condition.

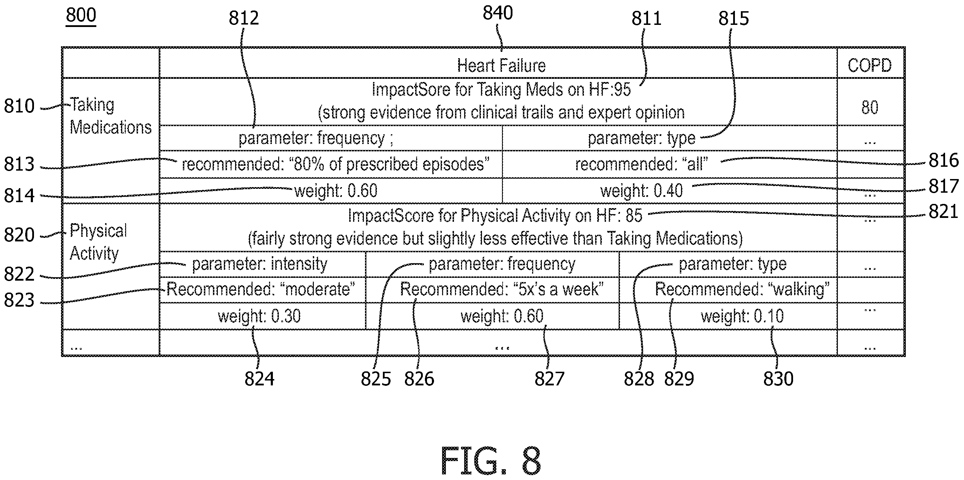

Figure 8 illustrates a portion of an exemplary database 800. Though Figure 8 illustrates the contents of the entries in the database 800 for two self-care behaviors and one medical condition, it will be apparent to those of skill in the art that a real-world implementation of a database 800 may include a larger variety of both self-care behaviors and medical conditions. In the database 800, entries for self-care behaviors 810 (in this example, taking medications) and 820 (in this example, physical activity) are provided for condition 840 (in this example, Heart Failure).

For self-care behavior 810, data included in the database includes ImpactScore 811, parameter 812 (in this example, frequency), recommended value 813 corresponding to parameter 812 (in this example, 80% of prescribed episodes, weight 814 corresponding to parameter 812, parameter 815 (in this example, type), recommended value 816 corresponding to parameter 815 (in this example, all), and weight 817 corresponding to parameter 815. For self-care behavior 820 (in this example, physical activity), data included in the database includes ImpactScore 821, parameter 822 (in this example, intensity), recommended value 823 corresponding to parameter 822 (in this example, moderate), weight 824 corresponding to parameter 822, parameter 825 (in this example, frequency), recommended value 826 corresponding to parameter 825 (in this example, five times per week), weight 827 corresponding to parameter 825, parameter 828 (in this example, type), recommended value 829 corresponding to parameter 828 (in this example, walking), and weight 830 corresponding to parameter 828. It will be apparent to those of skill in the art that the specific examples shown in Figure 8 are only exemplary and that these may vary for differing embodiments of a database 800.

In step 620, the Matcher 520 receives the patient profile from the Profiler 510 and data relevant to the patient's condition from the Database 530. This may be prompted by action by a clinician or by the patient or in any other appropriate manner. In step 625, the Matcher 520 generates an ordered list of self-care behaviors for the patient's medical condition. The ordered list may be ordered by the ImpactScore of the behaviors corresponding to the patient's condition. At this point in the method 600, the ImpactScores are still generalized based on the assumption that the patient carries out self-care behaviors in the optimal manner prescribed by clinical guidelines, and are not specific to the patient; in subsequent steps, the Matcher 520 will calculate updated ImpactScores that are specific to the patient.

In step 630, the Matcher 520 scales the ImpactScores of the behaviors contained in the ordered list based on the patient's current level of performance and the corresponding parameters. In this step, an upper bound for each parameter is defined as the recommended level, as determined by clinical guidelines, and assigned a numeric value of 100. (It will be apparent to those of skill in the art that the scaling value of 100 used here is only exemplary and that the following may be performed in the same manner with a different scaling value.) A lower bound for each parameter is defined as non-performance and assigned a numeric value of zero. The patient's current level of performance is then mapped to this 0-100 scale. This process is performed for each parameter. Considering physical activity as an example, if the recommended level of physical activity is five times per week, this is defined as the upper bound and assigned a numeric value of 100. Zero times per week is identified as the lower bound and assigned a numeric value of zero. If the data received from the Profiler 510 indicates that the patient performs physical activity once per week, this may be determined to have a numeric value of 20.

In step 635, the Matcher 520 customizes the values of some or all of the parameters based on the patient profile received from the Profiler 510. In particular, based on elements of the patient's self-care profile (e.g., as described in the self-assessment 40), certain types of behavior may be more or less effective. For example, physical activity can have many types, including walking on a treadmill at home, participating in a group exercise class, etc. A patient may choose one type of physical activity, and its associated value may be used in calculation of the ImpactScore for the physical activity. If each type of physical activity is effective as any other, then each may have the same numerical value. However, in some cases, one type of physical activity may be less effective than another due to the patient's self-care barriers; for example, a patient with a low level of social support or a low willingness to self-manage may be less effective at exercising at home than at exercising in a group exercise class where their exercise can be managed by an instructor and social support can be received from the instructor and classmates. Thus, in step 635, the values of various types of physical activity (and other self-care behaviors) may be adjusted based on the patient's self-care profile.

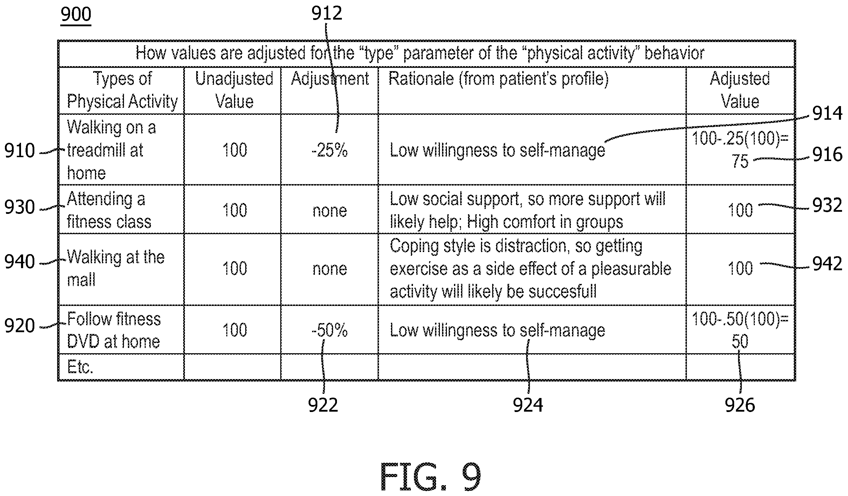

Figure 9 illustrates a table 900 showing the adjustment of the value of patient self-care behaviors based on the patient's self-management abilities. Self-care behavior 910 (in this example, walking on a treadmill at home) receives an adjustment 912 of -25% due to rationale 914 of the patient's low willingness to self-manage, resulting in an adjusted value 916 of 75. Similarly, self-care behavior 920 (in this example, following a fitness DVD at home) receives an adjustment 922 of -50% due to rationale 924 of the patient's low willingness to self-manage, resulting in adjusted value 926 of 50. Conversely, self-care behaviors 930 and 940 are not impacted by the patient's willingness to self-manage and, thus, are not adjusted due to this rationale, resulting in corresponding adjusted values 932 and 942, respectively, of 100. It will be apparent to those of skill in the art that the specific parameters used in such a table 900 may vary for different patients, different conditions, etc., and that the specific illustration of table 900 shown in Figure 9 is only exemplary.

In step 640, the Matcher 520 calculates adjusted, patient-specific ImpactScores for activities present in the ordered list. These revised ImpactScores are calculated so as to match the configuration and parameters specified by the patient and the patient's self-care profile. To do this calculation, a scaling factor is used. This scaling factor is calculated as:

$$ScalingFactor_{bm} = \sum (weight_i \times parameter_i)\,/100 \quad \forall\, i = 1, \ldots, \#parameters$$

The scaling factor is then used to calculate adjusted ImpactScores based on the following:

$$\text{ImpactScore}'_{bm} = \text{ScalingFactor}_{bm} * \text{ImpactScore}_{bm}$$

In the above formulas, $ImpactScore_{bm}$ refers to the effect that self-care behavior b, when optimally configured, has on medical condition m; $ImpactScore'_{bm}$ refers to the updated effect that self-care behavior b, as configured by the patient, has on medical condition *m*; $ScalingFactor_{bm}$ refers to the multiplier that scales the ImpactScore up or down based on the current configuration of the behavior *b*; $parameter_i$ is the value of the ith parameter, on a range from zero to 100; and $weight_i$ is a weight, ranging from 0 to 1, associated with the ith parameter, where the sum of all weights $weight_i$ is equal to 1.

Based on this model, the modification of a parameter value (equivalent to performing the corresponding self-care behavior more or less frequently, intensely, completely, etc.) will change the updated value ImpactScore' by an amount proportional to the weight of the parameter. Separating the parameters while maintaining their relationship to the overall updated value ImpactScore' is useful for helping the patient explore the relationship between the manner in which they carry out a self-care behavior and the effect the behavior will have on their clinical outcomes.

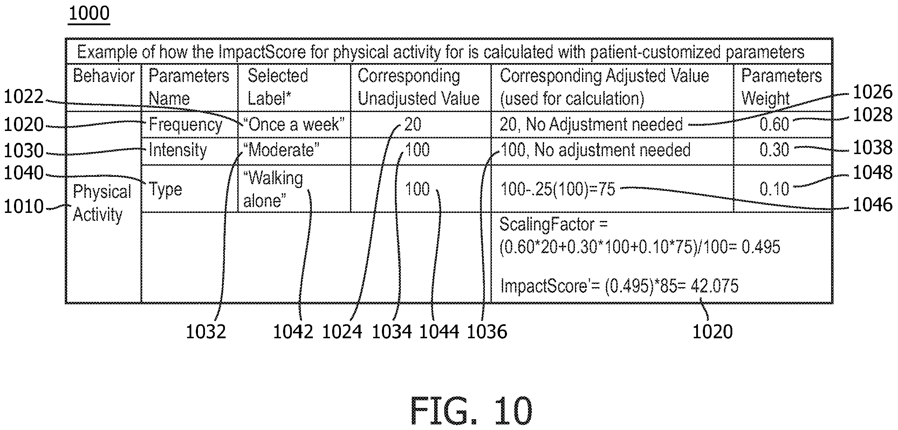

Figure 10 illustrates a table 1000 showing how an updated ImpactScore' for a given self-care behavior is determined using patient-customized parameters. For self-care behavior 1010 (in this example, physical activity), the patient responds to parameters 1020, 1030 and 1040 with parameter values 1022, 1032 and 1042 (in this example, once a week, moderate intensity, and walking alone, respectively). The parameters 1020, 1030 and 1040 have corresponding unadjusted values 1024, 1034 and 1044 (in this example, 20, 100 and 100, respectively). Values 1024 and 1034 have unchanged adjusted values 1026 and 1036 (in this example, 20 and 100, respectively), but value 1044 has a changed adjusted value 1046 (in this example, 75), for the reasons discussed above with reference to Figure 9. The parameters 1020, 1030 and 1040 also have corresponding parameter weights 1028, 1038 and 1048 (in this example, 0.60, 0.30 and 0.10, respectively). An updated ImpactScore' value 1050 is then calculated as described above and as illustrated in table 1000. It will be apparent to those of skill in the art that the specific values shown in table 1000 are only exemplary and that these may vary among different implementations, for different patients, for different medical conditions, etc.

In step 645, the Matcher 520 generates a patient-specific data model 540 for use by User Interface Application 550. The data model 540 may include any data necessary for the User Interface Application 550 to receive, from a user (e.g., the patient) one or more changes to parameters of the patient's self-care behaviors, and calculate an updated version of the updated, patient-specific $ImpactScore'_{bm}$ based on those changes using the formulas described above. The data model 540 may contain only data relevant to the patient's self-care profile, medical condition, and relevant self-care behaviors and their corresponding parameters, in order to be provided to the user for interactive use through User Interface Application 550 in a comparatively small data footprint (e.g., as compared to the Database 530 containing data for a wide variety of medical conditions and the Matcher 520 capable of processing a larger quantity of data) that may be appropriate for use on an end user device such as a tablet, mobile phone, personal computer, notebook computer, desktop computer, etc. Thus, the User Interface Application 550 may use the data model 540 to calculate an updated ImpactScore for the patient without communication with other elements of the system 500, such as the Matcher 520 or Database 530.

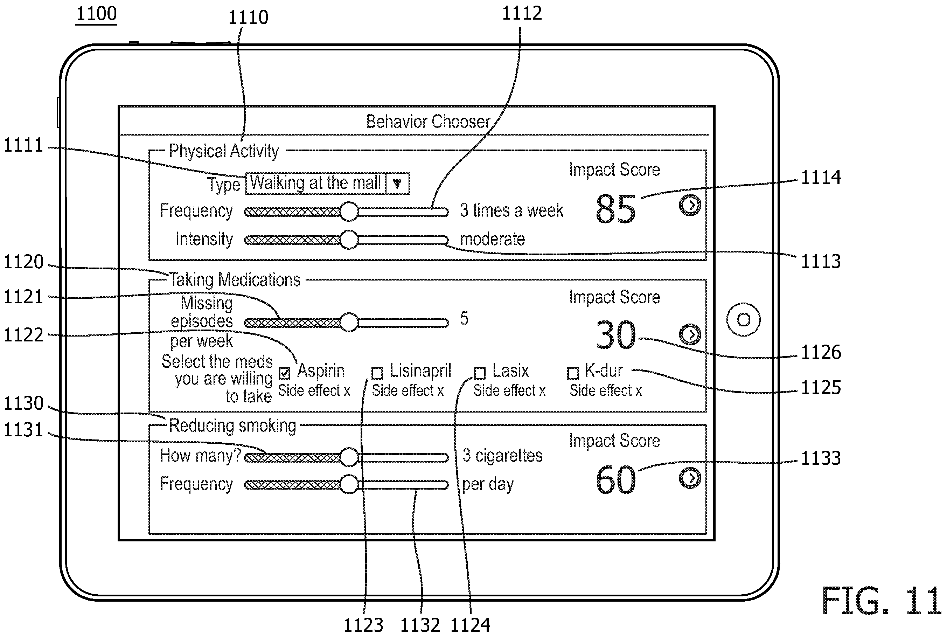

In step 650, the User Interface Application 550 presents, to a user (e.g., the patient) a display showing the user's self-care activities and the corresponding parameters for the self-care activities in an interactive manner allowing the user to vary the parameters (e.g., using known interface elements such as dropdown menus, sliders, checkboxes, etc.). Figure 11 illustrates an exemplary User Interface Application 550. In Figure 11, the User Interface Application 550 provides a display 1100 on a tablet device 1105, but other types of devices may execute the User Interface Application 550 and receive user input in a manner appropriate for the nature of the other devices. For example, for tablet device 1105 or a mobile phone, input may be received via a touchscreen; for a personal computer, input may be received with a mouse and/or keyboard; etc.

For the patient corresponding to the User Interface Application 550 as shown in Figure 9, the display 1100 includes three self-care behaviors 1110, 1120 and 1130. In the display 1100 shown in Figure 9, self-care behavior 1110 is physical activity, self-care behavior 1120 is taking medication, and self-care behavior 1130 is reducing smoking, but the specific self-care behaviors displayed by User Interface Application 550 may vary depending on the patient's medical condition. For each of the self-care behaviors 1110, 1120 and 1130, the display 1100 includes interface elements for corresponding parameters.

For self-care behavior 1110, which is physical activity, the display 1100 includes a dropdown menu 1111 for the user to select a type of physical activity (e.g., walking outside, walking at the mall, group exercise class, etc.). The display 1100 also includes a slider 1112 for the user to select a frequency for performing the selected physical activity. The display 1100 also includes a slider 1113 for the user to select an intensity for the selected physical activity. The display 1100 also includes an ImpactScore 1114 describing the level of effectiveness of the physical activity based on the user inputs 1111, 1112 and 1113, which may be determined as described above. It will be apparent to those of skill in the art that the ImpactScore 1114 and the other ImpactScores discussed hereinafter may be patient-specific adjusted ImpactScores determined as discussed above.

For self-care behavior 1120, which is taking medications, the display 1100 includes a slider 1121 for the user to select a number of episodes of missed medication per week. The display 1100 also includes checkboxes 1122, 1123, 1124 and 1125 for the user to select medications he/she is willing to take. The display 1100 also includes an ImpactScore 1126 describing the level of effectiveness of the user's medication based on the user inputs 1121, 1122, 1123, 1124 and 1125, which may be determined as described above.

For self-care behavior 1130, which is reducing smoking, the display 1100 includes a slider 1131 for the user to select a number of cigarettes smoked. The display 1100 also includes a slider 1132 for the user to select a frequency time period during which the user will smoke the number of cigarettes indicated by the slider 1131. The display 1100 also includes an ImpactScore 1133 describing the level of effectiveness of the user's smoking reduction based on the user inputs 1131 and 1132, which may be determined as described above. In one exemplary embodiment, the Impact-Scores 1114, 1126 and 1133 may be color-coded to more clearly indicate the level of effectiveness of the corresponding self-care behaviors 1110, 1120 and 1130. For example, an ImpactScore of 0 to 35 may be colored red, an ImpactScore of 36 to 70 may be colored yellow, and an ImpactScore of 71 to 100 may be colored green.

In step 655, the User Interface Application 550 waits for changed input parameters from the user. As described above, changed input parameters may mean adjusting a dropdown menu, a slider, a checkbox, etc. If changed input parameters are received by the User Interface Application 550, then, in step 660, the User Interface Application 550 calculates updated ImpactScore values (e.g., ImpactScores 1114, 1126 and 1133 of Figure 11) based on the newly-received parameters and the data model 540. After this, the method 600 returns to step 650, where the display 1100 is updated and the new results are displayed to the user. If no new parameters are received in step 655, then the method 600 proceeds to step 665, where the results currently displayed may be considered to be final results for the user's selected parameters. Following step 665, the method 600 terminates.

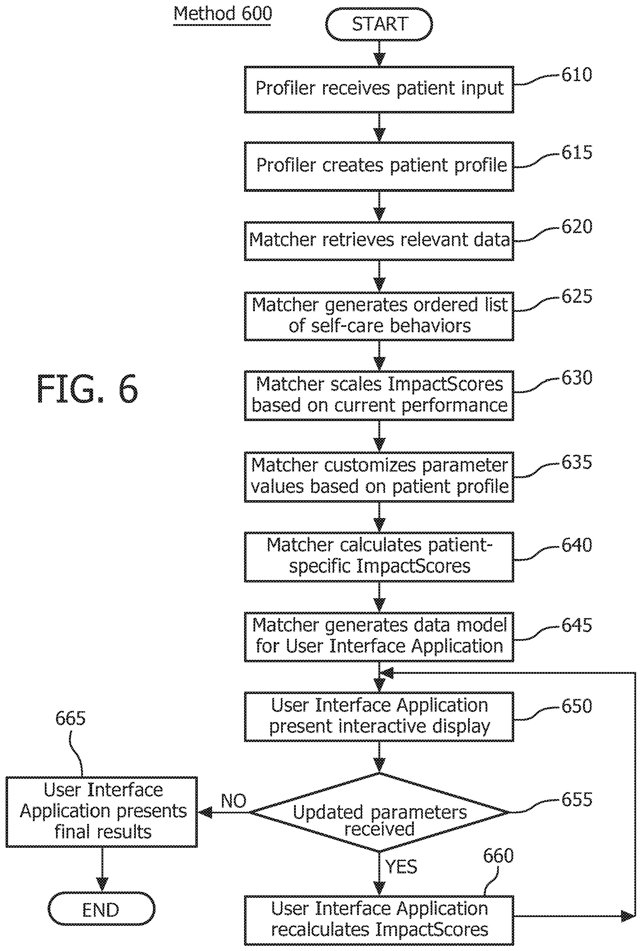

The exemplary embodiments of Figures 5-11 may provide users with the ability to visualize and dynamically modify parameters of their self-care behaviors, in order to understand the way those behaviors affect their clinical outcomes. This may be useful for patients across a wide spectrum of needs. In chronic disease management, where the trajectory of diseases is well established and the effects of self-care behaviors have been well-studied, this may be particularly useful. However, the same technique and interface may be equally applicable for choosing any health-related behavior. Some examples of this may include selection of exercise routines for general fitness purposes, selection of nutrition plans for dieting, selection of different medications, etc.

Those of skill in the art will understand that the above-described exemplary embodiments may be implemented in any number of manners, including as a software module, as a combination of hardware and software, etc. For example, the exemplary methods 200 and 600 may be embodied in a program stored in a non-transitory storage medium and containing lines of code that, when compiled, may be executed by a processor.

It will be apparent to those skilled in the art that various modifications may be made to the exemplary embodiments, without departing from the scope of the invention. Thus, it is intended that the present invention cover modifications and variations of this invention provided they come within the scope of the appended claims and their equivalents.

**Claims**

1. A non-transitory computer-readable storage medium storing a set of instructions executable by a processor, the set of instructions, when executed by the processor, causing the processor to perform operations comprising:

   receiving (220) a self-care assessment from a patient having a medical condition, the self-care assessment assessing the patient's level of ability for each of a plurality of determinants, wherein each determinant is a factor that may affect the patient's ability to adhere to self-care behaviors relevant to the medical condition;
   assigning (230) a weight to each of the plurality of determinants based on the patient's level of ability for each of the plurality of determinants;
   creating (240) an ordered list including the plurality of determinants, the determinants being ordered based on the corresponding weights;
   determining a plurality of behavior-specific contributions, each of the behavior-specific contributions corresponding to the contribution of each determinant to a care plan for a particular one of the plurality of self-care behaviors, wherein each behavior-specific contribution is based on the weight for the relevant determinant and a weight for the relevant self-care behavior; and
   designing a care plan for the patient based on the plurality of behavior-specific contributions.

2. The non-transitory computer-readable storage medium of claim 1, wherein the self-care assessment is one of a Self-Management Assessment questionnaire and a Patient Activation Measure questionnaire.

3. The non-transitory computer-readable storage medium of claim 1, further comprising:
   populating a matrix with the plurality of behavior-specific contributions.

4. The non-transitory computer-readable storage medium of claim 1, wherein the self-care behaviors include one or more of medication taking, symptom management, physical activity, nutrition, and smoking cessation.

5. The non-transitory computer-readable storage medium of claim 1, wherein the operations further comprise:
   receiving, from a clinician, a selection of a subset of the determinants, wherein only the selected subset of the determinants are included in the ordered list.

6. The non-transitory computer-readable storage medium of claim 1, wherein determinants assigned a lowest available weight are not included in the ordered list.

7. The non-transitory computer-readable storage medium of claim 1, wherein the weight of each of the plurality of determinants is calculated according to
$$W_D\left(D_{\mathrm{mod}}(i)\right) = \frac{weight\left(D_{\mathrm{mod}}(i)\right)}{\sum weight\left(D_{\mathrm{mod}}(i)\right)},$$

   wherein i ranges from 1 to a quantity of determinants,
   weight ($D_{\mathrm{mod}}(i)$) is an unscaled weight of each of the determinants, and $W_D(D_{\mathrm{mod}}(i))$ is the weight of each of the determinants.

8. The non-transitory computer-readable storage medium of claim 1, wherein each of the behavior-specific contributions is calculated according to $c(i,j)=W_D(D_{\mathrm{mod}}(i)) * W_B(j)$, wherein i ranges from 1 to a quantity of determinants, j ranges from 1 to a quantity of self-care behaviors, $W_D(D_{\mathrm{mod}}(i))$ is the weight of each of the determinants, $W_B(j)$ is the weight corresponding to each of the self-care behaviors, and c(i,j) is the one of the behavior-specific contributions corresponding to determinant i and self-care behavior j.

9. A non-transitory computer-readable storage medium storing a set of instructions executable by a processor, the set of instructions, when executed by the processor, causing the processor to perform operations comprising:

   receiving (610) a self-care assessment from a patient having a medical condition, the self-care assessment assessing the patient's level of ability for each of a plurality of determinants, wherein each determinant is a factor that may affect the patient's ability to adhere to self-care behaviors relevant to the medical condition;
   receiving (620), from a self-care database, a plurality of self-care behaviors relating to the medical condition, each of the self-care behaviors including a plurality of parameters relating to how each self-care behavior is performed, each of the parameters having a recommended value, each of the self-care behaviors also including a generalized, i.e. a non patient-specific, impact score corresponding to the medical condition, each generalized impact score quantifying (640) an effect that the self-care behavior has on the medical condition;

adjusting one or more of the generalized impact scores based on the levels of ability for one or more of the determinants to determine a plurality of patient-specific impact scores;

receiving (655) a patient input for each of the parameters;

determining (660) a plurality of adjusted impact scores based on the patient-specific impact scores and the patient inputs, each adjusted impact score quantifying an effect that the self-care behavior with the parameters according to the patient input will have on the medical condition; and

providing (665) a graphical user interface, wherein the patient input is received via the graphical user interface and the adjusted impact scores are provided via the graphical user interface.

10. The non-transitory computer-readable storage medium of claim 9, wherein the self-care behaviors include one or more of medication taking, symptom management, physical activity, nutrition, and smoking cessation.

11. The non-transitory computer-readable storage medium of claim 9, wherein the graphical user interface is provided to a user device with a user-specific data model, the user-specific data model including a subset of data from the self-care database that is relevant to the patient.

12. The non-transitory computer-readable storage medium of claim 9, wherein one of the parameters is one of a type, a frequency, and an intensity.

13. The non-transitory computer-readable storage medium of claim 9, wherein each of the adjusted impact scores is determined according to $ImpactScore'_{bm} = ScalingFactor_{bm} * ImpactScore_{bm}$, wherein $ImpactScore_{bm}$ is an impact score corresponding to the effect that self-care behavior b, when optimally configured, has on medical condition $m$; $ScalingFactor_{bm}$ is a scaling factor corresponding to the patient-specific impact scores and the patient inputs; and $ImpactScore'_{bm}$ is an impact score corresponding to the effect that self-care behavior $b$, has on medical condition $m$ in the case of the patient.

14. The non-transitory computer-readable storage medium of claim 13, wherein each of the plurality of scaling factors $ScalingFactor_{bm}$ is determined according to

$$ScalingFactor_{bm} = \sum (weight_i \times parameter_i)/100 \quad \forall\, i = 1, \ldots, \#parameters\,,$$

wherein $parameter_i$ is a value of the ith parameter in a range from zero to 100, and $weight_i$ is a weight, ranging from 0 to 1, associated with the ith parameter, wherein each value $weight_i$ is in a range from zero to 1, wherein the sum of all weights $weight_i$ is equal to 1.

15. The non-transitory computer-readable storage medium of claim 9, wherein each of the impact scores is represented on a scale ranging from -100 to 100.

**Patentansprüche**

1. Nicht-flüchtiges computerlesbares Speichermedium, in dem ein Satz von durch einen Prozessor ausführbaren Befehlen gespeichert ist, wobei der Satz von Befehlen bei Ausführung durch den Prozessor den Prozessor veranlasst, Vorgänge durchzuführen, die Folgendes umfassen:

Empfangen (220) einer Selbstpflegebewertung von einem Patienten mit einer Erkrankung, wobei die Selbstpflegebewertung den Grad der Fähigkeit des Patienten für jede von einer Vielzahl von Determinanten bewertet, wobei jede Determinante ein Faktor ist, der die Fähigkeit des Patienten zur Einhaltung von Selbstpflegeverhaltensweisen bezüglich der Erkrankung beeinträchtigen kann;

Zuweisen (230) eines Gewichts zu jeder der Vielzahl von Determinanten basierend auf dem Grad der Fähigkeit des Patienten für jede der Vielzahl von Determinanten;

Erstellen (240) einer sortierten Liste, die die Vielzahl von Determinanten einschließt, wobei die Determinanten basierend auf den entsprechenden Gewichten sortiert sind;

Bestimmen einer Vielzahl von verhaltensspezifischen Beiträgen, wobei jeder der verhaltensspezifischen Beiträge dem Beitrag jeder Determinante zu einem Pflegeplan für eine bestimmte der Vielzahl von Selbstpflegeverhaltensweisen entspricht, wobei jeder verhaltensspezifische Beitrag auf dem Gewicht für die relevante Determinante und einem Gewicht für das relevante Selbstpflegeverhalten basiert; und

Entwerfen eines Pflegeplans für den Patienten basierend auf der Vielzahl von verhaltensspezifischen Beiträgen.

2. Nicht-flüchtiges computerlesbares Speichermedium nach Anspruch 1, wobei die Selbstpflegebewertung eines von einem Self-Management Assessment-Fragebogen und einem Patient Activation Measure-Fragebogen ist.

3. Nicht-flüchtiges computerlesbares Speichermedium nach Anspruch 1, ferner umfassend:
Bevölkern einer Matrix mit der Vielzahl von verhaltensspezifischen Beiträgen.

4. Nicht-flüchtiges computerlesbares Speichermedium nach Anspruch 1, wobei die Selbstpflegeverhaltensweisen eines oder mehrere von Einnahme der Medikamente, Symptom-Management, körperliche Aktivität, Ernährung und Einstellen des Rauchens umfassen.

5. Nicht-flüchtiges computerlesbares Speichermedium nach Anspruch 1, wobei die Vorgänge ferner Folgendes umfassen:
Empfangen, von einem Arzt, einer Auswahl einer Teilmenge der Determinanten, wobei nur die ausgewählte Teilmenge der Determinanten in die sortierte Liste aufgenommen wird.

6. Nicht-flüchtiges computerlesbares Speichermedium nach Anspruch 1, wobei Determinanten, denen ein niedrigstes verfügbares Gewicht zugewiesen ist, nicht in die sortierte Liste aufgenommen werden.

7. Nicht-flüchtiges computerlesbares Speichermedium nach Anspruch 1, wobei das Gewicht von jeder der Vielzahl

$$W_D(D_{mod}(i)) = \frac{Gewicht\,(D_{mod}(i))}{\sum Gewicht\,(D_{mod}(i))}$$

von Determinanten gemäß berechnet wird, wobei $i$ von 1 bis zu einer Quantität der Determinanten reicht, *Gewicht ($D_{mod}(i)$)* ein unskaliertes Gewicht von jeder der Determinanten ist und $W_D$ *($D_{mod}(i)$)* das Gewicht von jeder der Determinanten ist.

8. Nicht-flüchtiges computerlesbares Speichermedium nach Anspruch 1, wobei jeder der verhaltensspezifischen Beiträge berechnet wird gemäß

$$c\,(i, j) = W_D\,(D_{mod}\,(i)) * W_B\,(j),$$

wobei $i$ von 1 bis zu einer Quantität der Determinanten reicht, $j$ von 1 bis zu einer Quantität von Selbstpflegeverhaltensweisen reicht, $W_D$ $(D_{mod}(i))$ das Gewicht von jeder der Determinanten ist, $W_B$ $(j)$ das Gewicht ist, das jeder der Selbstpflegeverhaltensweisen entspricht, und $c$ $(i, j)$ der eine der verhaltensspezifischen Beiträge ist, der der Determinante $i$ und der Selbstpflegeverhaltensweise $j$ entspricht.

9. Nicht-flüchtiges computerlesbares Speichermedium, in dem ein Satz von durch einen Prozessor ausführbaren Befehlen gespeichert ist, wobei der Satz von Befehlen bei Ausführung durch den Prozessor den Prozessor veranlasst, Vorgänge durchzuführen, die Folgendes umfassen:

Empfangen (610) einer Selbstpflegebewertung von einem Patienten mit einer Erkrankung, wobei die Selbstpflegebewertung den Grad der Fähigkeit des Patienten für jede von einer Vielzahl von Determinanten bewertet, wobei jede Determinante ein Faktor ist, der die Fähigkeit des Patienten zur Einhaltung von Selbstpflegeverhaltensweisen bezüglich der Erkrankung beeinträchtigen kann;

Empfangen (620), von einer Selbstpflegedatenbank, einer Vielzahl von Selbstpflegeverhaltensweisen in Bezug auf die Erkrankung, wobei jede der Selbstpflegeverhaltensweisen eine Vielzahl von Parametern in Bezug darauf einschließt, wie jede Selbstpflegeverhaltensweise durchzuführen ist, wobei jeder der Parameter einen empfohlenen Wert hat, wobei jede der Selbstpflegeverhaltensweisen auch eine generalisierte, d. h. eine nicht-patientenspezifische, Auswirkungspunktzahl einschließt, die der Erkrankung entspricht, wobei jede generalisierte Auswirkungspunktzahl eine Wirkung quantifiziert (640), die die Selbstpflegeverhaltensweise auf die Erkrankung hat;

Anpassen von einer oder mehreren der generalisierten Auswirkungspunktzahlen basierend auf dem Grad der Fähigkeit für eine oder mehrere der Determinanten, um eine Vielzahl von patientenspezifischen Auswirkungspunktzahlen zu bestimmen:

Empfangen (655) einer Patienteneingabe für jeden der Parameter;
Bestimmen (660) einer Vielzahl von angepassten Auswirkungspunktzahlen basierend auf den patientenspezifischen Auswirkungspunktzahlen und den Patienteneingaben, wobei jede angepasste Auswirkungspunktzahl eine Wirkung quantifiziert, die die Selbstpflegeverhaltensweise mit den Parametern gemäß der Patienteneingabe auf die Erkrankung haben wird; und
Bereitstellen (665) einer grafischen Benutzeroberfläche, wobei die Patienteneingabe über die grafische Benutzeroberfläche empfangen wird und die angepassten Auswirkungspunktzahlen über die grafische Benutzeroberfläche bereitgestellt werden.

10. Nicht-flüchtiges computerlesbares Speichermedium nach Anspruch 9, wobei die Selbstpflegeverhaltensweisen eines oder mehrere von Einnahme der Medikamente, Symptom-Management, körperliche Aktivität, Ernährung und Einstellen des Rauchens umfassen.

11. Nicht-flüchtiges computerlesbares Speichermedium nach Anspruch 9, wobei die grafische Benutzeroberfläche für eine Benutzervorrichtung mit einem benutzerspezifischen Datenmodell bereitgestellt wird, wobei das benutzerspezifische Datenmodell eine Teilmenge von Daten aus der Selbstpflegedatenbank umfasst, die für den Patienten relevant ist.

12. Nicht-flüchtiges computerlesbares Speichermedium nach Anspruch 9, wobei einer der Parameter eines von einem Typ, einer Häufigkeit und einer Intensität ist.

13. Nicht-flüchtiges computerlesbares Speichermedium nach Anspruch 9, wobei jede der angepassten Auswirkungspunktzahlen gemäß Auswirkungspunktzahl'$_{bm}$ = Skalierungsfaktor$_{bm}$ * Auswirkungspunktzahl$_{bm}$ bestimmt wird, wobei Auswirkungspunktzahl$_{bm}$ eine Auswirkungspunktzahl ist, die der Wirkung entspricht, die die Selbstpflegeverhaltensweise $b$, wenn sie optimal konfiguriert ist, auf eine Erkrankung $m$ hat; Skalierungsfaktor$_{bm}$ ein Skalierungsfaktor ist, der den patientenspezifischen Auswirkungspunktzahlen und Patienteneingaben entspricht; und Auswirkungspunktzahl'$_{bm}$ eine Auswirkungspunktzahl ist, die der Wirkung entspricht, die die Selbstpflegeverhaltensweise $b$ im Fall des Patienten auf die Erkrankung $m$ hat.

14. Nicht-flüchtiges computerlesbares Speichermedium nach Anspruch 13, wobei jeder der Vielzahl von Skalierungsfaktoren Skalierungsfaktor$_{bm}$ bestimmt wird gemäß Skalierungsfaktor$_{bm}$ = $\sum$ (Gewicht$_i$ x Parameter$_i$)/100 $\forall$ i = 1, ..., # Parameter, wobei Parameter$_i$ ein Wert des i-ten Parameters in einem Bereich von null bis 100 ist, und Gewichti ein Gewicht von 0 bis 1 ist, das dem i-ten Parameter zugehörig ist, wobei jeder Wert Gewichti in einem Bereich von null bis 1 liegt, wobei die Summe aller Gewichte Gewicht$_i$ gleich 1 ist.

15. Nicht-flüchtiges computerlesbares Speichermedium nach Anspruch 9, wobei jede der Auswirkungspunktzahlen auf einer Skala von -100 bis 100 dargestellt wird.

**Revendications**

1. Support de stockage non transitoire lisible par ordinateur stockant un ensemble d'instructions exécutables par un processeur, l'ensemble d'instructions, lorsqu'il est exécuté par le processeur, amenant le processeur à effectuer des opérations comprenant :

la réception (220) d'une évaluation de soins autonomes d'un patient ayant une condition médicale, l'évaluation de soins autonomes évaluant le niveau de capacité du patient pour chacun d'une pluralité de déterminants, dans lequel chaque déterminant est un facteur qui peut affecter la capacité du patient à adhérer aux comportements de soins autonomes connexes à la condition médicale ;
l'affectation (230) d'un poids à chacun de la pluralité de déterminants sur la base du niveau de capacité du patient pour chacun de la pluralité de déterminants ;
la création (240) d'une liste ordonnée incluant la pluralité de déterminants, les déterminants étant ordonnés sur la base des poids correspondants ;
la détermination d'une pluralité de contributions spécifiques aux comportements, chacune des contributions spécifiques aux comportements correspondant à la contribution de chaque déterminant à un plan de soins pour l'un particulier de la pluralité de comportements de soins autonomes, dans lequel chaque contribution spécifique à un comportement est basé sur le poids du déterminant connexe et un poids pour le comportement de soins autonomes connexe ; et

la conception d'un plan de soins pour le patient sur la base de la pluralité de contributions spécifiques aux comportements.

2. Support de stockage non transitoire lisible par ordinateur selon la revendication 1, dans lequel l'évaluation de soins autonomes est l'un parmi un questionnaire d'évaluation d'autogestion et un questionnaire de mesure d'activation de patients.

3. Support de stockage non transitoire lisible par ordinateur selon la revendication 1, comprenant en outre :
le peuplement d'une matrice avec la pluralité de contributions spécifiques aux comportements.

4. Support de stockage non transitoire lisible par ordinateur selon la revendication 1, dans lequel les comportements de soins autonomes comprennent un ou plusieurs des suivants : prise de médicaments, gestion de symptômes, activité physique, nutrition et cessation de l'usage du tabac.

5. Support de stockage non transitoire lisible par ordinateur selon la revendication 1, dans lequel les opérations comprennent en outre :
la réception par un clinicien d'une sélection d'un sous-ensemble des déterminants, dans lequel seul le sous-ensemble sélectionné des déterminants est inclus dans la liste ordonnée.

6. Support de stockage non transitoire lisible par ordinateur selon la revendication 1, dans lequel les déterminants auxquels est affecté un poids disponible le plus bas ne sont pas inclus dans la liste ordonnée.

7. Support de stockage non transitoire lisible par ordinateur selon la revendication 1, dans lequel le poids de chacun

$$W_D\big(D_{\mathrm{mod}}(i)\big) = \frac{weight\big(D_{\mathrm{mod}}(i)\big)}{\sum weight\big(D_{\mathrm{mod}}(i)\big)},$$

de la pluralité de déterminants est calculé selon la formule dans laquelle i se situe dans une plage de 1 à une quantité de déterminants, le poids ($D_{mod}(i)$) est un poids non à l'échelle de chacun des déterminants et $W_D(D_{mod}(i))$ est le poids de chacun des déterminants.

8. Support de stockage non transitoire lisible par ordinateur selon la revendication 1, dans lequel chacune des contributions spécifiques aux comportements est calculée selon la formule $c(i,j) = W_D(D_{mod}(i))*W_B(j)$, dans laquelle i se situe dans une plage de 1 à une quantité de déterminants, j se situe dans une plage de 1 à une quantité de comportements de soins autonomes, $W_D(D_{mod}(i))$ est le poids de chacun des déterminants, $W_B(J)$ est le poids correspondant à chacun des comportements de soins autonomes et c(i,j) est celle de contributions spécifiques aux comportements correspondant au déterminant i et au comportement de soins autonomes j.

9. Support de stockage non transitoire lisible par ordinateur stockant un ensemble d'instructions exécutables par un processeur, l'ensemble d'instructions, lorsqu'il est exécuté par le processeur, amenant le processeur à effectuer les opérations comprenant :

la réception (610) d'une évaluation de soins autonomes d'un patient ayant une condition médicale, l'évaluation de soins autonomes évaluant le niveau de capacité du patient pour chacun d'une pluralité de déterminants, dans lequel chaque déterminant est un facteur qui peut affecter la capacité du patient à adhérer aux comportements de soins autonomes connexes à la condition médicale ;
la réception (620) à partir d'une base de données de soins autonomes d'une pluralité de comportements de soins autonomes connexes à la condition médicale, chacun des comportements de soins autonomes incluant une pluralité de paramètres en rapport avec la manière dont chaque comportement de soins autonomes est effectué, chacun des paramètres ayant une valeur recommandée, chacun des comportements de soins autonomes incluant également un score d'impact généralisé, c'est-à-dire non spécifique au patient, correspondant à la condition médicale, chaque score d'impact généralisé quantifiant (640) un effet que le comportement de soins autonomes a sur la condition médicale ;
l'ajustement d'un ou plusieurs scores d'impact généralisés sur la base des niveaux de capacité pour un ou plusieurs des déterminants à déterminer une pluralité de scores d'impact spécifiques aux patients ;
la réception (655) d'une entrée de patient pour chacun des paramètres ;
la détermination (660) d'une pluralité de scores d'impact ajustés basés sur les scores d'impact spécifiques aux patients et sur les entrées de patients, chaque score d'impact ajusté quantifiant un effet que le comportement de soins autonomes avec les paramètres selon l'entrée de patient aura sur la condition médicale ; et

la fourniture (665) d'une interface utilisateur graphique, dans lequel l'entrée de patient est reçue via l'interface utilisateur graphique et les scores d'impact ajustés sont fournis via l'interface utilisateur graphique.

10. Support de stockage non transitoire lisible par ordinateur selon la revendication 9, dans lequel les comportements de soins autonomes comprennent un ou plusieurs des suivants : prise de médicaments, gestion de symptômes, activité physique, nutrition et cessation de l'usage de tabac.

11. Support de stockage non transitoire lisible par ordinateur selon la revendication 9, dans lequel l'interface utilisateur graphique est fournie à un dispositif utilisateur avec un modèle de données spécifiques à l'utilisateur, le modèle de données spécifiques à l'utilisateur incluant un sous-ensemble de données de la base de données d'autogestion qui est connexe au patient.

12. Support de stockage non transitoire lisible par ordinateur selon la revendication 9, dans lequel l'un des paramètres est l'un(e) parmi un type, une fréquence et une intensité.

13. Support de stockage non transitoire lisible par ordinateur selon la revendication 9, dans lequel chacun des scores d'impact ajustés est déterminé selon l'équation $ImpactScore'_{bm} = ScalingFactor_{bm} * ImpactScore_{bm}$, où $ImpactScore_{bm}$ est un score d'impact correspondant à l'effet que le comportement de soins autonomes b, lorsqu'il est configuré de manière optimale, a sur la condition médicale $m$ ; $ScalingFactor_{bm}$ est un facteur d'échelle correspondant aux scores d'impact spécifiques aux patients et aux entrées de patients ; et $ImpactScore'_{bm}$ est un score d'impact correspondant à l'effet que le comportement de soins autonomes $b$ a sur la condition médicale $m$ dans le cas du patient.

14. Support de stockage non transitoire lisible par ordinateur selon la revendication 13, dans lequel chacun de la pluralité de facteurs d'échelle $ScalingFactor_{bm}$ est déterminé selon l'équation

$$ScalingFactor_{bm} = \sum (weight_i \times parameter_i)/100 \quad \forall\, i = 1, ..., \#parameters,$$

dans lequel $parameter_i$ est une valeur du ième paramètre dans une plage de zéro à 100 et $weight_i$ est un poids allant de 0 à 1, associé au ième paramètre, dans lequel chaque valeur $weight_i$ se situe dans une plage de zéro à 1, dans lequel la somme de tous les poids $weight_i$ est égale à 1.

15. Support de stockage non transitoire lisible par ordinateur selon la revendication 9, dans lequel chacun des scores d'impact est représenté sur une échelle allant de -100 à 100.

```
┌─────────────────────────┐              ┌─────────────────────┐
│  Clinician's Choice of  │─20           │   Patient Self-     │─40
│      Determinants       │              │    Assessment       │
└─────────────────────────┘              └─────────────────────┘
           │                                        │
100        │                                        │
   ┌───────┼────────────────────────────────────────┼───────────┐
   │ System│                                        │           │
   │       ▼                                        ▼           │
   │  ┌──────────────────────────────────────────────────┐      │
   │  │                                                   │──110 │
   │  │                      Triage                       │      │
   │  │                                                   │      │
   │  └──────────────────────────────────────────────────┘      │
   │                         │                                  │
   │                         ▼                                  │
   │  ┌──────────────────────────────────────────────────┐      │
   │  │                                                   │──120 │
   │  │                   Ordered List                    │      │
   │  │                                                   │      │
   │  └──────────────────────────────────────────────────┘      │
   │                         │                                  │
   │                         ▼                                  │
   │  ┌──────────────────────────────────────────────────┐      │
   │  │                                                   │──130 │
   │  │                    Quantifier                     │      │
   │  │                                                   │      │
   │  └──────────────────────────────────────────────────┘      │
   └─────────────────────────┼──────────────────────────────────┘
                             ▼
      ┌──────────────────────────────────────────────────┐
      │                                                   │──140
      │               Contributions Matrix                │
      │                                                   │
      └──────────────────────────────────────────────────┘
```

FIG. 1

Method 200

START

Triage receives clinician's
choice of determinants — 210

Triage receives patient
selfassessment data — 220

Triage assigns weight to each
modifiable determinant — 230

Triage creates ordered
list of determinants — 240

Triage sends ordered list
of determinants to Quantifier — 250

Quantifier calculates a weight
for each determinant — 260

Quantifier determines
contributions matrix — 270

END

FIG. 2

| Sub-set of determinants (Dmod) based on clinician's choice | Assessment Scores | Weights w1>w2>w3 | Weights Instances Example A | Weights Instances Example B |
|---|---|---|---|---|
| -disease burden | High<br>Average<br>Low | w1<br>w2<br>w3 | 1<br>2<br>3 | 0<br>1<br>2 |
| -perceived control over health | Self-controlled<br>Controlled by circumstances<br>Controlled by others | w1<br>w2<br>w3 | 1<br>2<br>3 | 0<br>1<br>2 |
| -self-efficacy | High<br>Average<br>Low | w1<br>w2<br>w3 | 1<br>2<br>3 | 0<br>1<br>2 |
| -social support | Enough support<br>some support<br>No support | w1<br>w2<br>w3 | 1<br>2<br>3 | 0<br>1<br>2 |
| -coping with problems | By solving problems<br>By expressing emotions<br>By seeking distraction | w1<br>w2<br>w3 | 1<br>2<br>3 | 0<br>1<br>2 |
| -willingness to self-management | High<br>Average<br>Low | w1<br>w2<br>w3 | 1<br>2<br>3 | 0<br>1<br>2 |

300  310  320  330  340  350

FIG. 3

EP 3 146 452 B1

410

| Dmod |
|---|
| -disease burden<br>-persceived control over health<br>-self-efficacy<br>-social support<br>-coping with problems<br>-willingness to self-management |

# FIG. 4A

420

●●● More suitable for
self-management

Perceived control over health
*Self-controlled*

self efficacy
*High*

willingness to self-manage
*High*

Social support
*Some support*

Confort in groups
*High*

Coping with problems
*By expressing emotions*

Computer akilla
*Low*

Anxiety
*Never*

Desease burden
*Low*

Depression
*Never*

# FIG. 4B

430

| Dmod | SeMaS assessment score | Weights |
|---|---|---|
| -disease burden | -low | 2 |
| -persceived control over health | -self-controlled | 0 |
| -self-efficacy | -high | 0 |
| -social support | -some support | 1 |
| -coping with problems | -by expressing emotions | 1 |
| -willingness to self-management | -high | 0 |

# FIG. 4C

450

| Ordered Dmod | weight(Dmod(i)) | Wd(Dmod(i)) |
|---|---|---|
| 1. desease burden | 2 | 2/4=1/2 (=50%) |
| 2. social support | 1 | 1/4 (=25%) |
| 3.coping with problems | 1 | 1/4 (=25%) |

# FIG. 4E

120

| # | Sub-set of determinants (Dmod) based on clinician's choice | Assessment scores | Weights Instances Example B |
|---|---|---|---|
| 1 | -disease burden | Low | 2 |
| 2 | -social support | Some support | 1 |
| 3 | -coping with problems | By expressing emotions | 1 |
| 4 | -perceived control over health | Self-controlled | 0 |
| 5 | -self-efficacy | High | 0 |
| 6 | -willingness to self-management | High | 0 |

**FIG. 4D**

460

| Ordered Dmod | Meds taking Wb(1) = 40% | Symp. Mng Wb(2) = 30% | PA | Nutrition Wb(i) = 10% i = 3,4,5 | Smoking Cessation |
|---|---|---|---|---|---|
| 1. desease burden | 1/2*40%=20% | 1/2*30%=15% | | 1/2*10%=5% | |
| 2. social support | 1/4*40%=10% | 1/4*30%=7.5% | | 1/4*10%=2.5% | |
| 3.coping with problems | 1/4*40%=10% | 1/4*30%=7.5% | | 1/4*10%=2.5% | |

**FIG. 4F**

EP 3 146 452 B1

140

| Dmod | 1. Medication Taking 40% | 2. Symptom Management 30% | 3. Physical Activity 10% | 4. Nutrition 10% | 5. Smoking Cessation 10% | Total |
|---|---|---|---|---|---|---|
| 1. Disease burden | 20% | 15% | 5% | 5% | 5% | 50% |
| 2. Social Support | 10% | 7.5% | 2.5% | 2.5% | 2.5% | 25% |
| 3. Coping with problems | 10% | 7.5% | 2.5% | 2.5% | 2.5% | 25% |
| 4. Perceived control | 0% | 0% | 0% | 0% | 0% | 0% |
| 5. Self-efficacy | 0% | 0% | 0% | 0% | 0% | 0% |
| 6. Will. to self-manage | 0% | 0% | 0% | 0% | 0% | 0% |
| Anxiety | | | | | | |
| Depression | | | | | | |
| Comfort in a group | | | | | | |
| Computer skills | | | | | | |

FIG. 4G

EP 3 146 452 B1

FIG. 5

Method 600

START

Profiler receives patient input — 610

Profiler creates patient profile — 615

Matcher retrieves relevant data — 620

Matcher generates ordered list of self-care behaviors — 625

**FIG. 6**

Matcher scales ImpactScores based on current performance — 630

Matcher customizes parameter values based on patient profile — 635

Matcher calculates patient-specific ImpactScores — 640

Matcher generates data model for User Interface Application — 645

User Interface Application present interactive display — 650

665

User Interface Application presents final results

NO ← Updated parameters received — 655

YES

660

User Interface Application recalculates ImpactScores

END

23

| Profile Element | Value | Type |
|---|---|---|
| Conditions | Heart Failure (severity:Class II); Diabetes (severity index: 1/low) | List of conditions |
| Current performance | physical Activity: walking alone at moderate intensity once a week; Medication Adherence: skips medications once week; Smoking: 5 cigarettes/day | List of behaviors and performance level |
| Social Support | Low | Scale (low, moderate,high) |
| Local of Control | External-others | Choice (internal, external-doctor, external-others) |
| Willingness to self-manage | Low | Scale (low, moderate,high) |
| Patient's prioritized outcome | Extending lifespan | Choice (Quality of Live, Reducing hospitalizations, Extending lifespan, Reducing costs) |
| etc. | ... | ... |

FIG. 7

EP 3 146 452 B1

| 800 | | Heart Failure | | | COPD |
|---|---|---|---|---|---|
| 810 Taking Medications | | ImpactSore for Taking Meds on HF:95 (strong evidence from clinical trails and expert opinion | | | 80 |
| | | parameter: frequency ; | parameter: type | | ... |
| 813 | | recommended: "80% of prescribed episodes" | recommended: "all" | | ... 816 |
| 814 | | weight: 0.60 | weight: 0.40 | | ... 817 |
| 820 Physical Activity | | ImpactScore for Physical Activity on HF: 85 (fairly strong evidence but slightly less effective than Taking Medications) | | | ... 821 |
| 822 | | parameter: intensity | parameter: frequency | parameter: type | ... |
| 823 | | Recommended: "moderate" | Recommended: "5x's a week" | Recommended: "walking" | |
| | | weight: 0.30 | weight: 0.60 | weight: 0.10 | ... |
| ... | | ... | | | ... |

812   840   811   815

824   825   826   827   828   829   830

FIG. 8

EP 3 146 452 B1

EP 3 146 452 B1

900

912

| How values are adjusted for the "type" parameter of the "physical activity" behavior | | | | |
|---|---|---|---|---|
| Types of Physical Activity | Unadjusted Value | Adjustment | Rationale (from patient's profile) | Adjusted Value |
| Walking on a treadmill at home | 100 | -25% | Low willingness to self-manage | 100-.25(100)= 75 |
| Attending a fitness class | 100 | none | Low social support, so more support will likely help; High comfort in groups | 100 |
| Walking at the mall | 100 | none | Coping style is distraction, so getting exercise as a side effect of a pleasurable activity will likely be succesfull | 100 |
| Follow fitness DVD at home | 100 | -50% | Low willingness to self-manage | 100-.50(100)= 50 |
| Etc. | | | | |

910  930  940  920

914  916  932  942

922  924  926

FIG. 9

## 1000

| Example of how the ImpactScore for physical activity for is calculated with patient-customized parameters | | | | | |
|---|---|---|---|---|---|
| Behavior | Parameters Name | Selected Label* | Corresponding Unadjusted Value | Corresponding Adjusted Value (used for calculation) | Parameters Weight |
| Physical Activity | Frequency | "Once a week" | 20 | 20, No Adjustment needed | 0.60 |
| | Intensity | "Moderate" | 100 | 100, No adjustment needed | 0.30 |
| | Type | "Walking alone" | 100 | 100-.25(100)=75 | 0.10 |
| | | | | ScalingFactor = (0.60*20+0.30*100+0.10*75)/100= 0.495  ImpactScore'= (0.495)*85= 42.075 | |

FIG. 10

EP 3 146 452 B1

FIG. 11

EP 3 146 452 B1

**EP 3 146 452 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013098719 A2